# EUROPEAN PATENT APPLICATION

(11) **EP 4 056 553 A1**
(43) Date of publication of application: **14.09.2022**
(21) Application number: 21161165.2
(22) Date of filing: 08.03.2021
(51) Int. Cl.: C07C 253/30, C07C 255/46

(54) **ALKYL SUBSTITUTED CYCLOHEXANECARBONITRILES**

(71) Applicant: Solvay SA, 1120 Brussels (BE)
(72) Inventor: LORENT, Karol, 7110 Strépy-Bracquegnies (BE); DABEUX, François, 1140 Evere (BE)
(74) Representative: Vande Gucht, Anne

(57) **Abstract**

The invention relates to specific alkyl substituted cyclohexanecarbonitriles having the Formula I 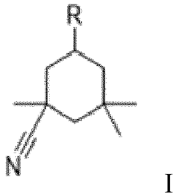 wherein R is an alkyl group comprising at least two carbon atoms, production processes thereof and their use as solvent in the manufacture of an aqueous hydrogen peroxide solution.

## Description

The present invention relates to specific alkyl substituted cyclohexanecarbonitriles, processes for manufacturing them and to their use as solvent in the manufacture of an aqueous hydrogen peroxide solution.

Hydrogen peroxide is one of the most important inorganic chemicals to be produced worldwide. Its industrial applications include textile, pulp and paper bleaching, organic synthesis (propylene oxide), the manufacture of inorganic chemicals and detergents, environmental and other applications.

Synthesis of hydrogen peroxide is predominantly achieved by using the Riedl-Pfleiderer process (originally disclosed in U.S. Pat. Nos. 2,158,525 and 2,215,883), also called anthraquinone loop process or AO (auto-oxidation) process.

This well-known cyclic process makes use typically of the auto-oxidation of at least one alkylanthrahydroquinone and/or of at least one tetrahydroalkylanthrahydroquinone, most often 2-alkylanthraquinone, to the corresponding alkylanthraquinone and/or tetrahydroalkylanthraquinone, which results in the production of hydrogen peroxide.

The first step of the AO process is the reduction in an organic solvent (generally a mixture of solvents) of the chosen quinone (alkylanthraquinone or tetrahydroalkylanthraquinone) into the corresponding hydroquinone (alkylanthrahydroquinone or tetrahydroalkylanthrahydroquinone) using hydrogen gas and a catalyst. The mixture of organic solvents, hydroquinone and quinone species (working solution, WS) is then separated from the catalyst and the hydroquinone is oxidized using oxygen, air or oxygen-enriched air thus regenerating the quinone with simultaneous formation of hydrogen peroxide.

The organic solvent of choice is typically a mixture of two types of solvents, one being a good solvent of the quinone derivative (generally a non-polar solvent for instance a mixture of aromatic compounds) and the other being a good solvent of the hydroquinone derivative (generally a polar solvent for instance a long chain alcohol or an ester).

Hydrogen peroxide is then typically extracted with water and recovered in the form of a crude aqueous hydrogen peroxide solution, and the quinone is returned to the hydrogenator to complete the loop.

The use of di-isobutyl-carbinol (DIBC) as polar solvent is namely described in Patent applications EP 529723, EP 965562 and EP 3052439 in the name of the Applicant. The use of a commercial mixture of aromatics sold under the brand Solvesso^{®}-150 (CAS no. 64742-94-5) as non-polar solvent is also described in said patent applications. This mixture of aromatics is also known as Caromax, Shellsol, A150, Hydrosol, Indusol, Solvantar, Solvarex and others, depending on the supplier. It can advantageously be used in combination with sextate (methyl cyclohexyl acetate) as polar solvent (see namely US Patent 3617219).

Most of the AO processes use either 2-amylanthraquinone (AQ), 2-butylanthraquinone (BQ) or 2-ethylanthraquinone (EQ). Especially in the case of EQ, the productivity of the working solution is limited by the lack of solubility of the reduced form of ETQ (ETQH). It is namely so that EQ is largely and relatively quickly transformed in ETQ (the corresponding tetrahydroalkylanthraquinone) in the process. Practically, that ETQ is hydrogenated in ETQH to provide H₂O₂ after oxidation. The quantity of EQH produced is marginal in regards of ETQH. It means that the productivity of the process is directly proportional to the amount of ETQH produced. The reasoning is the same for a process working with AQ or BQ instead of EQ.

The hydrogenated quinone solubility issue is known from prior art and some attempts were made to solve it. Namely co-pending PCT application EP2019/056761 to the Applicant, discloses the use of non-aromatic cyclic nitrile type solvents as polar solvent in the mixture, more specifically the use of cyclohexanecarbonitriles, and especially substituted ones (in which the nitrile function is protected from chemical degradation).

Although some molecules of this kind are known, their market availability is currently on very limited and any too small to satisfy the needs of an industrial AO process.

Furthermore, some of the known syntheses require a relatively large number of reaction steps and are thus less interesting for industrial manufacturing processes. For instance, 2,2,6-trimethylcyclohexanecarbonitrile was synthesized by Shive et al. (JACS, 1942, vol. 64, pp.385-389) starting from geranic acid which was first cyclized using formic acid (1); was then hydrogenated to the corresponding saturated acid (2,2,6-trimethylcyclohexanecarboxylic acid) (2), which was then transformed in the corresponding acyl chloride using thionyl chloride (3), then in the corresponding amide using ammonia (4) and finally, in the corresponding carbonitrile by dehydration using phosphorus pentoxide (5). Hence, this way of synthesis implies 5 reaction steps.

Additionally, the alkyl substituted cyclohexanecarbonitrile generated by this process only has 10 C atoms (hence, is a C10) while we found out that better results in terms of hydrogenated quinone solubility and insolubility in H₂O₂ (hence higher purity level of the H2O2) can be obtained with nitriles having a higher number of C atoms i.e. C11, C12 or C12+ (i.e. more than 12C atoms). In particular, nitriles having 12 or more carbon atoms have up till now not been reported in literature.

Therefore, in a first aspect, the present invention relates to specific alkyl substituted cyclohexanecarbonitriles having 12 or more carbon atoms. In particular, the invention relates to a compound having the Formula I 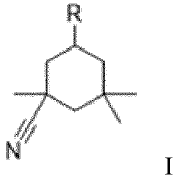 wherein R is an alkyl group comprising at least 2 carbon atoms.

In a preferred embodiment, R of Formula I is an alkyl group comprising at least 3, at least 4, at least 5 or more preferably at least 6 C atoms. It is further preferred that the number of C atoms of the alkyl group of the compound of Formula I is between 2 and 20 C atoms, 3 to 18 C atoms, 4 to 16 C atoms, 5 to 14 C atoms, more preferably between 6 to 12 C atoms.

The alkyl group of the compound of Formula I can be a linear, a branched or a cyclo-alkyl group. It is particularly preferred that alkyl group of the compound of Formula I is selected from the group consisting of ethyl, propyl, butyl, pentyl, hexyl, cyclohexyl and isomers thereof.

It is even more preferred that the compound of Formula I is selected from the group consisting of:
5-ethyl-1,3,3-trimethylcyclohexanecarbonitrile (C12B),
1,3,3-trimethyl-5-propylcyclohexanecarbonitrile (C13A),
1,3,3-trimethyl-5-isopropylcyclohexanecarbonitrile (C13B),
5-(*n*-butyl)-1,3,3-trimethylcyclohexanecarbonitrile (C14A),
5-(2-butyl)-1,3,3-trimethylcyclohexanecarbonitrile (C14B),
1,3,3-trimethyl-5-(n-pentyl)cyclohexanecarbonitrile (C15A),
1,3,3-trimethyl-5-(2-pentyl)cyclohexanecarbonitrile (C15B),
1,3,3-trimethyl-5-(3-pentyl)cyclohexanecarbonitrile (C15C),
5-(*n*-hexyl)-1,3,3-trimethylcyclohexanecarbonitrile (C16A),
5-(2-hexyl)-1,3,3-trimethylcyclohexanecarbonitrile (C16B),
5-(3-hexyl)-1,3,3-trimethylcyclohexanecarbonitrile (C16C) and
5-cyclohexyl-1,3,3-trimethylcyclohexanecarbonitrile (C16D).

In a particular preferred embodiment, the compound of Formula I is 5-ethyl-1,3,3-trimethylcyclohexanecarbonitrile (C12B).

A second aspect of the invention is to provide a production process for alkyl substituted cyclohexanecarbonitriles having 12 or more carbon atoms which is simple and uses inexpensive and/or environmental friendly raw materials and thus the process is suitable for industrial processes. Such a process has up till now not been reported in literature.

Therefore, according to the invention, as starting material α, β-unsaturated cyclohexanone 3,3,5-trimethylhex-2-enone (also called isophorone) or cyanoisophorone (also called isophorone nitrile or IPN) is used. Isophorone and IPN are cheap and widely available compounds.

Isophorone is produced on a multi-thousand ton scale by basic or acid catalysed condensation of acetone.

Cyanation of isophorone to obtain IPN has been reported in literature like for instance in the article to Dischino et al. in the Journal of Labelled Compounds and Radiopharmaceuticals, 42, 965-974 (1999), in US4299775 and in WO 2011/095576. It generally involves the use of compounds like KCN, NaCN, CuCN, Zn(CN)₂, AlEt₂CN and the like. KCN and/or NaCN are preferred for an industrial process mainly for economic reasons.

Cyanation preferably takes place in a polar solvent like DMF, DMSO or sulfolane. The reaction temperature preferably is from 50 to 150°C, preferably between 100 and 140°C, most preferably about 120°C. The reaction generally happens at a pressure from atmospheric pressure up till 10 bar, most preferably at atmospheric pressure and until full conversion is reached. Alternatively, and even more preferably especially in case AlEt₂CN is used, cyanation can take place in a non-polar organic solvent like toluene, at lower temperature, typically from -10°C until ambient temperature, preferably between 0 and 20°C.

After cyanation of the isophorone, in order to obtain the compound of Formula I according to the invention, a Horner-Wadsworth-Emmons (HWE) reaction and subsequently a hydrogenation reaction can be conducted. In the HWE reaction a triphenyl phosphonium salt Ph₃PR⁺ X⁻ is used, wherein R is an alkyl group as defined with respect to compound of Formula I and X is a halogen ion, preferably I, Cl or Br, to transform the carbonyl group of IPN into the corresponding alkenyl group.

This type of reaction is also called Wittig reaction. Usually in a Wittig reaction a triphenyl phosphonium salt is deprotonated with strong base, such as for example potassium *tert*-butoxide, *n*-butyllithium or sodium amide, to obtain the corresponding alkenyl-triphenyl-phosphorane.

Subsequently, according to the invention, the alkenyl-triphenyl-phosphorane reacts with the carbonyl group of IPN which results in the corresponding alkenyl substituted cyclohexanecarbonitrile. The reaction preferably takes place in an ether solvent such as for example THF or methyl THF.

Afterwards, the alkenyl substituted cyclohexanecarbonitrile is hydrogenated to obtain the compound of Formula I. The hydrogenation process can be conducted in any way as known in the art, for example as described below.

However, even though an alkyl substituted cyclohexanecarbonitrile according to the invention can be obtained by the above described synthesis, in order to further simplify the manufacturing process it is preferred to obtain a compound according to Formula I by a manufacturing process, which comprises the following steps:
- reaction of cyanoisophorone (IPN) with an organometallic compound comprising an alkyl group (R) as defined above in order to transform the ketone moiety into its corresponding alcohol;
- reduction of the alcohol in order to obtain the alkyl substituted cyclohexanecarbonitrile of the invention.

In a preferred embodiment, said process comprising the additional step of synthesizing the cyanoisophorone through cyanation of isophorone as described above.

The organometallic compound that can be used to transform the ketone group into a ternary alcohol can be a Grignard reagent, i.e. an organo-Mg compound. A Grignard reagent is preferred, more particularly alkylMgBr or alkylMgCl, wherein the alkyl group is identical to the alkyl group as defined with respect to the compound of Formula I. The reaction preferably takes place in an ether solvent (THF or methyl THF for instance), in anhydrous conditions. The reaction temperature preferably is below 0°C, more preferably below -5°C, - 10 °C, more preferably the reaction temperature is about -30 °C or about -25 °C. The reaction is advantageously performed at atmospheric pressure.

The step of reducing the alcohol in order to obtain the alkyl substituted cyclohexanecarbonitrile of the invention preferably uses an organosilane reducing agent like Et₃SiH, AlCl₃, TiCl₄ or Boron trifluoride etherate (strictly boron trifluoride diethyl etherate, or boron trifluoride-ether complex) preferably assisted by TFA (TriFluoroAcetic Acid). The reaction temperature preferably is from 20 to 100°C, more preferably from 30 to 80°C, most preferably about 50°C. The reaction if preferably conducted at atmospheric pressure and can be conducted solvent free or in a polar solvent like DCM or DCE.

Another possibility, according to the invention, to reduce the alcohol in order to obtain the compound of Formula I is to react in a first step the 1,3,3-trimethyl-5-alky-5-hydroxy-cyclohexylcarbonitrile with phosphorous tribromide (PBr₂), phosphorous trichloride (PCl₃), phosphorus triiodide (PI₃), potassium iodide (KI) with acid catalysis, thionyl chloride (SOCl₂) or thionyl bromide (SOBr₂), preferably in the presence of a compound able to trap the acid released (e.g. HCl) like pyridine, trimethylamine, DIPEA (Hunig's base), proton sponge, imidazole, or any aromatics containing a pyridine-like nitrogen able to react with the acid to give the corresponding salt, inorganic bases like Na₂CO₃, sodium bicarbonate etc., and preferably in the presence of an inert solvent, like toluene. In a second step, a hydrogenation reaction is conducted to obtain the alkyl substituted cyclohexylcarbonitrile of the invention. The hydrogenation reaction can be carried out in any way known in the art, preferably the hydrogenation reaction takes place in the presence of a hydrogenation catalyst for instance based on Ni, Pd or Pt. Each of these metal catalysts is preferably prepared in a special way and/or in a given form:
- nickel is usually used in a finely divided form called "Raney nickel" and which is prepared by reacting a Ni-Al alloy with NaOH;
- palladium is generally obtained commercially "supported" on an inert substance, such as charcoal (i.e. as a Pd/C catalyst) and ethanol is generally chosen as solvent in this case;
- platinum is generally used as PtO₂, also called Adams' catalyst, although it is actually platinum metal that is the catalyst; the hydrogen used to add to the carbon-carbon double bond also reduces the platinum(IV) oxide to finely divided platinum metal. Ethanol or acetic acid is generally used as solvent with this catalyst.

Good results are obtained in the frame of the invention with PtO₂ (i.e. the Adams' catalyst) as catalyst and acetic acid (preferably glacial acetic acid) as solvent. Hydrogenation preferably takes place at a temperature from ambient to 100°C, preferably from 30 to 80°C, more preferably from 40 to 60°C, a temperature about 50°C or about 40 °C giving good results in practice. Hydrogenation preferably takes place at a pressure from atmospheric to 20 bar, the higher the substitution degree the higher the pressure.

According to the invention, it is preferred that the compounds of Formulas C12B, C13A, C13B, C14A, C14B, C15A, C15B, C15C, C16A, C16B, C16C and C16D are obtained by the above described processes, even more it is preferred that the compound of Formula C12B is obtained by the above described processes.

Finally, the present invention also relates to the use of the compound of Formula I as a polar organic solvent in a process for manufacturing hydrogen peroxide comprising the following steps:
- hydrogenating a working solution which comprises an alkylanthraquinone and/or tetrahydroalkylanthraquinone and a mixture of a non-polar organic solvent and the alkyl substituted cyclohexanecarbonitrile of the invention as polar organic solvent;
- oxidizing the hydrogenated working solution to produce hydrogen peroxide; and
- isolating the hydrogen peroxide.

The term "alkylanthraquinone" is intended to denote a 9,10-anthraquinone substituted in position 1, 2 or 3 with at least one alkyl side chain of linear or branched aliphatic type comprising at least one carbon atom. Usually, these alkyl chains comprise less than 9 carbon atoms and, preferably, less than 6 carbon atoms. Examples of such alkylanthraquinones are ethylanthraquinones like 2-ethylanthraquinone (EQ), 2-*iso*propylanthraquinone, 2*-sec-* and 2*-tert-*butylanthraquinone (BQ), 1,3-, 2,3-, 1,4- and 2,7-dimethylanthraquinone, amylanthraquinones (AQ) like 2*-iso-* and 2-*tert*-amylanthraquinone and mixtures of these quinones.

The term "tetrahydroalkylanthraquinone" is intended to denote the 9,10-tetrahydroquinones corresponding to the 9,10-alkylanthraquinones specified above. Hence, for EQ and AQ, they respectively are designated by ETQ and ATQ, their reduced forms (tetrahydroalkylanthrahydroquinones) being respectively ETQH and ATQH.

Preferably, an AQ or EQ is used, the latter being preferred.

In order to be able to also solubilize the quinone, the polarity of the solvent mixture is preferably not too high. Hence, there is preferably at least 30wt% of non-polar solvent in the organic solvents mixture, and more preferably at least 40wt%. Generally, there is not more than 80wt% of this non-polar solvent, preferably not more than 60wt% of it in the organic solvents mixture.

The non-polar solvent preferably is an aromatic solvent or a mixture of aromatic solvents. Aromatic solvents are for instance selected from benzene, toluene, xylene, *tert*-butylbenzene, trimethylbenzene, tetramethylbenzene, naphthalene, methylnaphthalene mixtures of polyalkylated benzenes, and mixtures thereof. The commercially available aromatic hydrocarbon solvent of type 150 from the Solvesso^{®} series (or equivalent from other supplier) gives good results. S-150 (Solvesso^{®}-150; CAS no. 64742-94-5) is known as an aromatic solvent of high aromatics which offer high solvency and controlled evaporation characteristics that make them excellent for use in many industrial applications and in particular as process fluids. The Solvesso^{®} aromatic hydrocarbons are available in three boiling ranges with varying volatility, e.g. with a distillation range of 165-181°C, of 182-207 °C or 232-295 °C. They may be obtained also naphthalene reduced or as ultra-low naphthalene grades. Solvesso^{®} 150 (S-150) is characterized as follows: distillation range of 182-207°C; flash point of 64 °C; aromatic content of greater than 99 % by wt; aniline point of 15 °C; density of 0.900 at 15 °C; and an evaporation rate (nButAc=100) of 5.3.

The hydrogenation reaction takes place in the presence of a catalyst (like for instance the one object of WO 2015/049327 in the name of the Applicant) and as for instance described in WO 2010/139728 also in the name of the applicant (the content of both references being incorporated by reference in the present application). Typically, the hydrogenation is conducted at a temperature of at least 45°C and preferably up to 120°C, more preferably up to 95°C or even up to 80°C only. Also typically, the hydrogenation is conducted at a pressure of from 0.2 to 5 bar. Hydrogen is typically fed into the vessel at a rate of from 650 to 750 normal m³ per ton of hydrogen peroxide to be produced.

The oxidation step may take place in a conventional manner as known for the AO-process. Typical oxidation reactors known for the anthraquinone cyclic process can be used for the oxidation. Bubble reactors, through which the oxygen-containing gas and the working solution are passed co-currently or counter-currently, are frequently used. The bubble reactors can be free from internal devices or preferably contain internal devices in the form of packing or sieve plates. Oxidation can be performed at a temperature in the range from 30 to 70° C, particularly at 40 to 60° C. Oxidation is normally performed with an excess of oxygen, so that preferably over 90%, particularly over 95%, of the alkyl anthrahydroquinones contained in the working solution in hydroquinone form are converted to the quinone form.

After the oxidation, during the purification step, the hydrogen peroxide formed is separated from the working solution generally by means of an extraction step, for example using water, the hydrogen peroxide being recovered in the form of a crude aqueous hydrogen peroxide solution. The working solution leaving the extraction step is then recycled into the hydrogenation step in order to recommence the hydrogen peroxide production cycle, eventually after having been treated/regenerated.

In a preferred embodiment, after its extraction, the crude aqueous hydrogen peroxide solution is washed several times i.e. at least two times consecutively or even more times as required to reduce the content of impurities at a desired level.

The term "washing" is intended to denote any treatment, which is well known in the chemical industry (as disclosed in GB841323A, 1956 (Laporte), for instance), of a crude aqueous hydrogen peroxide solution with an organic solvent which is intended to reduce the content of impurities in the aqueous hydrogen peroxide solution. This washing can consist, for example, in extracting impurities in the crude aqueous hydrogen peroxide solution by means of an organic solvent in apparatuses such as centrifugal extractors or liquid/liquid extraction columns, for example, operating counter-current wise. Liquid/liquid extraction columns are preferred. Among the liquid/liquid extraction columns, columns with random or structured packing (like Pall rings for instance) or perforated plates are preferred. The former are especially preferred.

In a preferred embodiment, a chelating agent can be added to the washing solvent in order to reduce the content of given metals. For instance, an organophosphorus chelating agent can be added to the organic solvent as described in the above captioned patent application EP 3052439 in the name of the Applicant, the content of which is incorporated by reference in the present application.

The expression "crude aqueous hydrogen peroxide solution" is intended to denote the solutions obtained directly from a hydrogen peroxide synthesis step or from a hydrogen peroxide extraction step or from a storage unit. The crude aqueous hydrogen peroxide solution can have undergone one or more treatments to separate out impurities prior to the washing operation according to the process of the invention. It typically has an H₂O₂ concentration within the range of 30-50% by weight.

The use of the alkyl substituted cyclohexanecarbonitrile of the invention as polar solvent makes it is possible to achieve a higher solubility of the hydrogenated quinone and thus there is less polar solvent needed to achieve a higher partition coefficient. With this higher partition coefficient it is possible to reduce the capex (capital expenditure) required for the extraction sector.

The solvent of the invention is particularly suitable for the manufacture of hydrogen peroxide by the AO-process wherein said process has a production capacity of hydrogen peroxide of up to 100 kilo tons per year (ktpa). Preferably said process is a small to medium scale AO-process operated with a production capacity of hydrogen peroxide of up to 50 kilo tons per year (ktpa), and more preferably with a production capacity of hydrogen peroxide of up to 35 kilo tons per year (ktpa), and in particular a production capacity of hydrogen peroxide of up to 20 kilo tons per year (ktpa). The dimension ktpa (kilo tons per annum) relates to metric tons.

A particular advantage of such a small to medium scale AO-process is that the hydrogen peroxide can be manufactured in a plant that may be located at any, even remote, industrial end user site and the solvents of the invention are therefore especially suitable. It is namely so that since their partition coefficient is more favourable, less emulsion is observed in the process and a purer H₂O₂ solution can be obtained (namely containing less TOC) and this for a longer period of time compared to when solvents known from prior art are used.

In a preferred sub-embodiment of the invention, the working solution is regenerated either continuously or intermittently, based on the results of a quality control, regeneration meaning conversion of certain degradates, like epoxy or anthrone derivatives, back into useful quinones. Here also, the solvents of the invention are favorable because the quality of the H₂O₂ solution can be maintained within the specifications namely in terms of TOC for a longer period of time.

## Claims

1. A compound of Formula I 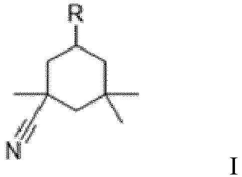 wherein R is an alkyl group comprising at least 2 carbon atoms.

2. A compound according to claim 1, wherein the alkyl group is a linear, branched or cyclo-alkyl group.

3. A compound according to claim 1 or 2, wherein the compound of Formula I is selected from the group consisting of:
5-ethyl-1,3,3-trimethylcyclohexanecarbonitrile (C12B),
1,3,3-trimethyl-5-propylcyclohexanecarbonitrile (C13A),
1,3,3-trimethyl-5-isopropylcyclohexanecarbonitrile (C13B),
5-(*n*-butyl)-1,3,3-trimethylcyclohexanecarbonitrile (C14A),
5-(2-butyl)-l,3,3-trimethylcyclohexanecarbonitrile (C14B),
1,3,3-trimethyl-5-(n-pentyl)cyclohexanecarbonitrile (C15A),
1,3,3-trimethyl-5-(2-pentyl)cyclohexanecarbonitrile (C15B),
1,3,3-trimethyl-S-(3-pentyl)cyclohexanecarbonitrile (C15C),
5-(*n*-hexyl)-1,3,3-trimethylcyclohexanecarbonitrile (C16A),
5-(2-hexyl)-1,3,3-trimethylcyclohexanecarbonitrile (C16B),
5-(3-hexyl)-1,3,3-trimethylcyclohexanecarbonitrile (C16C) and
5-cyclohexyl-1,3,3-trimethylcyclohexanecarbonitrile (C16D).

4. A process for the manufacturing of compound according to any one of claims 1-3, comprising the following steps:
- reaction of cyanoisophorone with a tri-phenyl phosphonium compound
Ph₃P R⁺ X⁻,
wherein R is an alkyl group comprising at least two carbon atoms and X is an halogen ion, in order to transform the carbonyl group of the cyanoisophorone to the corresponding alkenyl group
and
- hydrogenation of the obtained alkenyl substituted cyclohexanecarbonitrile to the compound according to any one of claims 1-3.

5. A process for the manufacturing of compound according to any one of claims 1-3, comprising the following steps:
- reaction of cyanoisophorone with an organometallic compound comprising an alkyl group comprising at least two carbon atoms, in order to transform the ketone moiety into its corresponding alcohol;
- reduction of the alcohol in order to obtain the compound according to any one of claims 1-3.

6. The process according to claim 4 or 5, wherein said process comprises the additional step of synthesizing the cyanoisophorone through cyanation of isophorone.

7. The process according to claim 6, wherein the cyanation uses KCN and/or NaCN.

8. The process according to claim 6 or 7, wherein the cyanation takes place in a polar solvent like DMF, DMSO or sulfolane.

9. The process according to any one of claims 5 to 8, wherein the organometallic compound is a Grignard reagent.

10. The process according to claim 9, wherein the organometallic compound is alkylMgBr or alkylMgCl.

11. The process according to any one of claims 5 to 10, wherein the reaction of the cyanoisophorone with the organometallic compound takes place in an ether solvent (THF or methyl THF for instance), in anhydrous conditions.

12. The process according to any one of claims 5 to 11, wherein the step of reducing the alcohol is conducted
- by using an organosilane reducing agent like Et₃SiH, AlCl₃, TiCl₄ or Boron trifluoride etherate (strictly boron trifluoride diethyl etherate, or boron trifluoride-ether complex) preferably assisted by TFA (TriFluoroAcetic Acid),
or
- by using a phosphorous tribromide (PBr₂), phosphorous trichloride (PCl₃), phosphorus triiodide (PI₃), potassium iodide (KI) with acid catalysis, thionyl chloride (SOCl₂) or thionyl bromide (SOBr₂) followed by a hydrogenation reaction.

13. Use of the compound according to any one of claims 1 to 3 as polar organic solvent in a process for the manufacture of hydrogen peroxide comprising the following steps:
- hydrogenating a working solution which comprises an alkylanthraquinone and/or tetrahydroalkylanthraquinone and a mixture of a non-polar organic solvent and the compound as defined in any one of claims 1 to 3 as polar organic solvent;
- oxidizing the hydrogenated working solution to produce hydrogen peroxide; and
- isolating the hydrogen peroxide.

14. The use according to claim 13, which results into a production capacity of hydrogen peroxide of up to 100 kilo tons per year.

15. The use according to claim 13 or 15, wherein the process for the manufacture of hydrogen peroxide being operated in a plant located at an industrial end user site.
